(19) Europäisches Patentamt

European Patent Office

Office européen des brevets

(11) **EP 0 762 924 B1**

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(21) Anmeldenummer: **95920735.8**

(22) Anmeldetag: **30.05.1995**

(51) Int. Cl.$^7$: **B01D 53/72**, A61L 2/20

(86) Internationale Anmeldenummer:
**PCT/DE95/00700**

(87) Internationale Veröffentlichungsnummer:
**WO 95/32788 (07.12.1995  Gazette 1995/52)**

(54) **VERFAHREN ZUM ENTSORGEN DES GASES ETHYLENOXID NACH ERFOLGTER BEGASUNG VON ZU STERILISIERENDEM GUT UND VORRICHTUNG HIERZU**

PROCESS AND DEVICE FOR DISPOSING OF ETHYLENE OXIDE GAS USED FOR STERILISATION PURPOSES

PROCEDE ET DISPOSITIF D'ELIMINATION D'OXYDE D'ETHYLENE GAZEUX UTILISE POUR STERILISER DES OBJETS

(84) Benannte Vertragsstaaten:
**AT BE CH DE DK ES FR GB GR IE IT LI LU NL PT SE**

(30) Priorität: **31.05.1994 DE 4419001**

(43) Veröffentlichungstag der Anmeldung:
**19.03.1997  Patentblatt 1997/12**

(73) Patentinhaber: **Drzevitzky, Bernd**
**D-68165 Mannheim (DE)**

(72) Erfinder:
• **DRZEVITZKY, Bernd**
**D-68165 Mannheim (DE)**

• **LEITZKE, Hartmut**
**D-69502 Hemsbach (DE)**
• **WALKE, Hans-Jürgen**
**D-61381 Friedrichsdorf (DE)**

(74) Vertreter:
**Mierswa, Klaus, Dipl.-Ing.**
**Friedrichstrasse 171**
**68199 Mannheim (DE)**

(56) Entgegenhaltungen:
EP-A- 0 129 986            DE-A- 1 925 412
DE-A- 2 814 132            DE-A- 3 047 938
DE-C- 4 117 306            DE-C- 4 138 321

**Beschreibung**

Technisches Gebiet:

Die Erfindung betrifft ein Verfahren zum Entsorgen des Gases Ethylenoxid nach erfolgter Begasung von zu sterilisierendem Gut innerhalb einer druckdichten Sterilisationskammer gemäß Anspruch 1 sowie eine Vorrichtung hierzu gemäß Anspruch 5.

Stand der Technik:

Zur Sterilisation hitzeempfindlicher Medikalgüter, wie Einwegartikel oder Verbundartikel, mit Plastikbestandteilen für Mehrfachnutzung werden Sterilisatoren eingesetzt, die mit dem Gas Ethylenoxid zur Sterilisation betrieben werden und die aus einer gasdicht abgeschlossenen Sterilisationskammer mit Kammergrößen zwischen 0,05 bis ca. 70 m$^3$ bestehen, in die das Sterlisiergut eingebracht wird. Nach Verschluß und Evakuierung der enthaltenen Luft wird das zu sterilisierende Gut in aller Regel mit Wasserdampf befeuchtet und danach mit gasförmigem Ethylenoxid bei einem Dampfdruck bis zu 50.000 Pascal für mehrere Stunden zur Abtötung von Keimen und Sporen begast. Der Absolutdruck in der Kammer setzt sich aus den Partialdrücken des Ethylenoxids, beigegebener Inertgase, wie Kohlendioxid oder Stickstoff, Wasserdampf und nicht evakuierter Restluft zusammen. Ethylenoxid ist bei Konzentrationen von 2,5% bis 100% ein hochexplosives und extrem gesundheitsschädliches Gas, weshalb es entsprechend den gesundheitlichen Vorschriften und der TA-Luft entsorgt werden muß.

Nach erfolgter Begasung wird die Sterilisationskammer evakuiert. Bei industriellen Großanlagen wird das Abgas in aller Regel entweder über Verbrennungsanlagen oder Gaswäscher geleitet und entfernt. Da normale Vakuumpumpen bis zu Absolutdrücken von ca. $p \approx 5000$ Pa effektiv arbeiten, enthält die Sterilisationskammer nach der Evakuierung und Ableitung des Ethylenoxids über die Verbrennungsanlage oder den Gaswäscher noch große Mengen an Ethylenoxid. Wird die Sterilisationskammer mit Frischluft belüftet, mischt sich das restliche Ethylenoxid mit der zugeführten Luft, wodurch nach Definition der TA-Luft Abgas entsteht, das ebenfalls entsorgt werden muß. Dazu wird die Sterilisationskammer erneut evakuiert und das Abgas wie geschildert entsorgt. Nach erneuter Frischluftzufrhr wird die Restkonzentration auf etwa 1/20 der Konzentration der vorangehenden Spülung verringert. Dieser Vorgang muß so oft wiederholt werden, bis in der Sterilisationskammer die Restkonzentration die gesetzlich zulässige Konzentration von Ethylenoxid sicher unterschreitet (TRKC*8 < 16 mg EO/m$^3$).

Demzufolge sind bei Berücksichtigung der einsetzenden Desorption des Sterilisierguts mindestens sechs Spülzyklen der Sterilisationskammer erforderlich. Während bei der ersten Evakuierung Abgas mit einer Konzentration an Ethylenoxid gefördert wird, das hochexplosiv ist, ist bei den nachfolgenden Entsorgungszyklen z.B. eine autotherme Verbrennung nicht mehr möglich. Wegen der geringen Restkonzentration von Ethylenoxid im Abgas zumindest in den letzten Spülgängen ist die Einhaltung der Forderungen der TA-Luft (<5mg/m$^3$) schwierig und sehr kostenintensiv, zumal die Entsorgungseinrichtungen auf Abgasströme mit extremen Konzentrationsunterschieden ausgelegt werden müssen (2000 g/m$^3$ >c>5mg/m$^3$).

Des weiteren ergeben sich Probleme der Arbeitssicherheit aus der Desorption des Ethylenoxids aus dem Sterilisiergut mit großen Halbwertszeiten. Selbst bei der Erhöhung der Anzahl der Spülzyklien übersteigt der Massenstrom des Ethylenoxids nach der 8. Spülung durch Desorption den der Vakuumpumpe. Daher werden die geforderten Restkonzentrationen des Ethylenoxids insbesondere bei gewerblichen Sterilisationsanlagen in aller Regel nicht erreicht, weil die Zeit für die Spülzyklen zu lang ist. Je nach Auslegung der Vakuumpumpen werden für einen Spülzyklus bis zu 40 Minuten benötigt.

Durch H.-J. Frohn in: WLB Wasser, Luft und Boden 1-2, 1989, Seiten 43-44, ist eine Ethylenoxidabscheidung aus kleineren Sterilisatoren als Absorptionsverfahren bekannt geworden, bei dem das gasförmige Ethylenoxid chemisch in ein Waschmittel gebunden wird. Durch die chemische Bindung und Umsetzung des Ethylenoxids zu Ethylenglykol bleibt ein großes treibendes Konzentrationsgefälle zwischen Gas- und Flüssigphase erhalten. Als Waschmittel wird verdünnte Schwefelsäure verwendet; mit dem Wasseranteil der Schwefelsäure setzt sich das Ethylenoxid zu Ethylenglykol um. Dabei nimmt die Schwefelsäure lediglich als Katalysator an der Reaktion teil, so daß die in ihrem Aufnahmevermögen erschöpfte Waschlösung aus einem Schwefelsäure-Ethylenglykol-Wassergemisch besteht. Das Waschmittel wird durch zwei hintereinandergeschaltete Füllkörperkolonnen dispergiert, in denen das Waschmittel am Kolonnenkopf aufgegeben wird und durch die Packung hindurchrieselt; das ethylenoxidhaltige Abgas strömt im Gegenstrom zum Waschmittel durch die Kolonnen. Dieses Verfahren arbeitet diskontinuierlich als Batch-Absorption, wobei das Waschmittel so lange im Kreislauf über die Kolonnen gefahren wird, bis ein Sättigungszustand erreicht ist, der den Wechsel des erschöpften Waschmittels erforderlich macht. Nachteilig bei diesem Verfahren ist prinzipiell die Umwälzung des Waschmittels; die Verweildauer des Abgases kann nicht beliebig gesteuert werden entsprechend der Ethylenoxid-Konzentration. Bei kleinen Vorlagebehältern sind überdies hohe Säurekonzentrationen notwendig, große Ethylenoxidmengen können damit nicht bewältigt werden.

Ein weiteres ähnliches Absorptionsverfahren ist das Verfahren nach Croll-Reynolds für Druck- und Vakuumkammern, bei dem nach der Absorption des Ethylenoxids nachfolgend eine Hydrolyse zu Ethylenglykol erfolgt.

Durch die DE 41 38 321 C1 ist ein Verfahren und eine Anlage zur Rückgewinnung von Ethylenoxid bekannt geworden, bei dem zumindest ein Teil des nach dem Sterilisierprozeß aus einer Sterilisierkammer abgezogenen Sterilisiergases mittels Ausgefrierung durch flüssigen Stickstoff abgeschieden wird. Der vom abgeschiedenen Sterilisiergas befreite Gasstrom wird in die Sterilisierkammer zurückgeführt, während gleichzeitig zumindest ein Teil des abgeschiedenen Sterilisiergases unter Reduzierung der Konzentration des Sterilisiergases in der Sterilisierkammer durch Zugabe von inertisierendem Gas ersetzt wird. Durch die zu verwendende extreme Kälte ist das Verfahren insgesamt sehr anfällig, wobei aus physikalischen Gründen im abgezogenen Gasstrom ein Rest an Ethylenoxid von 2g pro m$^3$ verbleibt, der nicht abgeschieden werden kann.

Durch die EP-A 0 129 986 ist ein Verfahren zm Auswaschen von Ethylenoxid aus einem Gasstrom mit einem Wäscher bekannt geworden, wie er üblicherweise nach dem System Gas-in-Wasch- oder Reaktionsflüssigkeit vielfältig verwendet wird. Das mit Ethylenoxid belastete Abgas wird on-line durch den Wäscher mit Schwefelsäure als Katalysator zur Wandlung von Ethylenoxid zu Ethylenglykol gefahren und das Restgas in die Umwelt abgeleitet. Aus Gründen der Gasdynamik sind auf diese Weise kaum Absenkungen der Restgehalte an Ethylenoxid unter 500 bis 600 mg/m$^3$ bei einmaligem Waschdurchlauf erzielbar.

Technische Aufgabe:

Der Erfindung liegt die Aufgabe zugrunde, das in Sterilisationskammern eingesetzte Ethylenoxid unter Nutzung seiner physikalischen und chemischen Eigenschaften im engen Zusammenwirken mit den physikalischen Eigenschaften des Sterilisationsprozesses und unter Vermeidung extrem explosionsgefährdeter Anlagenteile sowie der Umwälzung des Waschmittels vollständig aus dem Abgas zu entfernen und in den Wertstoff Ethylenglykol umzuwandeln und gleichzeitig den Prozeßablauf der Abgasentsorgung zeitlich erheblich zu verkürzen und die Abgasmengen zu verringern.

Der Zweck der Erfindung besteht darin, das extrem umweltbelastende und hochexplosive Ethylenoxid nach Gebrauch bei einer Sterilisation vollständig am Ort des Gebrauchs aus dem Kammergas zu entfernen und in einen Wertstoff umzuwandeln.

Offenbarung der Erfindung und deren Vorteile:

Die Lösung der Aufgabe besteht erfindungsgemäß darin, daß zum Entsorgen des Gases Ethylenoxid nach erfolgter Begasung von zu sterilisierendem Gut innerhalb einer druckdicht verschließbaren Sterilisationskammer, unter Verwendung einer an die Sterilisationskammer angeschlossenen Pumpe, die das Ethylenoxid nach der Begasung einem mit Säure arbeitenden Gaswäscher zuführt, in welchem das Ethylenoxid in Ethylenglykol umgewandelt wird, Sterilisationskammer, Pumpe und Gaswäscher einen geschlossenen Gaskreislauf für das Ethylenoxidgas bilden, innerhalb des Gaskreislaufes die Pumpe das Ethylenoxid aus der Sterilisationskammer kontinuierlich dem Gaswäscher zuführt, dessen Abgase zurück der Sterilisationskammer aufgegeben werden, die über eine Kammerheizung beheizt wird, wobei das im Gaswäscher entstehende Ethylenglykol kontinuierlich oder diskontinuierlich aus dem Gaswäscher abgezogen und dafür dem Gaswäscher entsprechend dem Verbrauch an Säure kontinuierlich oder diskontinuierlich weitere Säure zugegeben wird und durch das Auswaschen des Ethylenoxids aus dem Kammergas und die Umwandlung desselben in Ethylenglykol der Innendruck in der Sterilisationskammer und im Gaskreislauf laufend vermindert wird, und daß dieser Gas-Kreisprozeß solange aufrechterhalten wird, bis der Ethylenoxidanteil des im Gaskreislauf verbliebenen umgewälzten Gases unter eine vorgegebene Konzentration gefallen ist und danach die Sterilisationskammer belüftet wird. Die Sterilisation innerhalb der Sterilisationskammer wird vorzugsweise mit einem Unterdruck gegenüber dem Athmosphärendruck betrieben, vorzugsweise mit einem Druck von 0,5 bar. Weitere vorteilhafte Ausgestaltungen der Erfindung sind in den Unteransprüchen gekennzeichnet.

Die Erfindung besitzt den Vorteil, daß aufgrund des Gas-Umwälzkreislaufes, in welchem sich die Sterilisationskammer befindet, nach der Begasung mit Ethylenoxid laufend das Ethylenoxid in einem Gaskreisprozeß dem umgewälzten Abgas entzogen und entsorgt wird, womit eine extreme Verkürzung der Ethylenoxidentsorgung gegeben ist. Ebenso können extrem kurze Leitungswege zwischen dem Anwendungsort der Sterilisation und dem Entsorgungsort des hochexplosiven und hochgiftigen Ethylenoxids geschaffen werden, weshalb das Gefahrenpotential des Ethylenoxids erheblich herabgesetzt wird. Des weiteren wird eine Verbrennung des Ethylenoxids nach der Begasung vollständig vermieden, so daß die beim Stand der Technik notwendige Energiezufuhr zur Verbrennung, die aufwendige Steuerung und Regelung der Prozesse sowie ein technisch aufwendiger Explosions- und Flammenrückschlagschutz entfallen. Ebenso wird aus dem Ethylenoxid der Wertstoff Ethylenglykol zurückgewonnen, aus welchem extern wiederum Ethylenoxid gewonnen werden kann. Insgesamt gesehen wird die Zeit für die Entsorgung des Ethylenoxid und des Ethylenglykols erheblich verkürzt.

In technischer Hinsicht können technisch aufwendige Drehschieberpumpen, die heute in Sterilisationsanlagen des Standes der Technik zum Einsatz gelangen, durch wartungsarme, einfache und betriebssichere Wasserringpumpen ersetzt werden.

Des weiteren können auch für kleine Sterilisationsanlagen nunmehr die gesetzlich vorgeschriebenen Grenzwerte laut Gefahrstoffverordnung bei der Kammerentleerung leicht und sicher eingehalten werden. Ebenso werden die Investitions- und Betriebskosten gewerblicher Sterilisationsanlagen beim Einsatz des erfindungsgemäßen Verfahrens erheblich verringert.

Da die Sterilisationskammer bei einem Druck vorzugsweise von 0,5 bar betrieben wird, ist nur das halbe Kammervolumen umzuwälzen gegenüber dem normalen Druck und anschließend als Abgas abzugeben. Gegenüber der katalytischen Verbrennung mit Frischluft bedeutet das eine ca 250-fache Gasvolumenverringerung, weil dort der Volumenstrom entsprechend verdünnt werden muß.

Kurzbeschreibung der Zeichnung, in der zeigen:

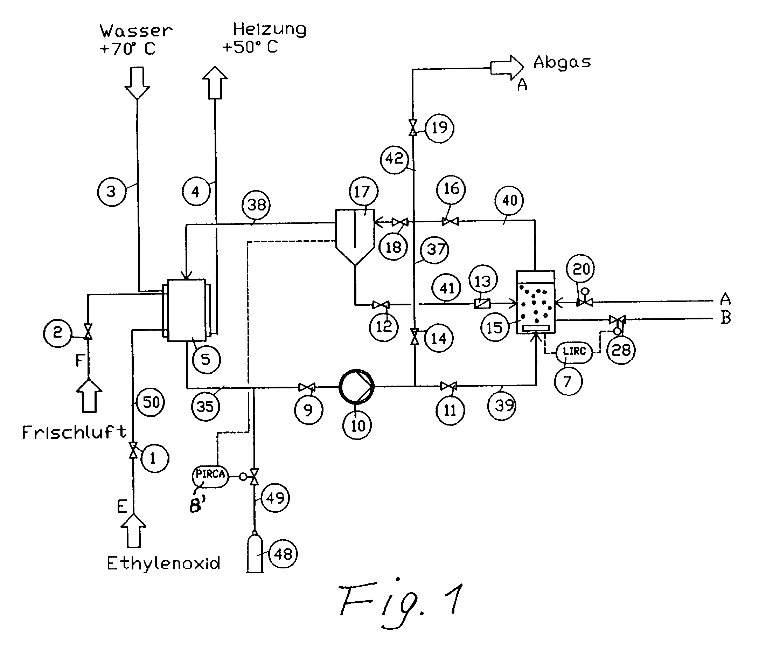

Figur 1      den ersten Teil eines Grundfließbildes der Vorrichtung bis zu den abgehenden Leitungen A-B und

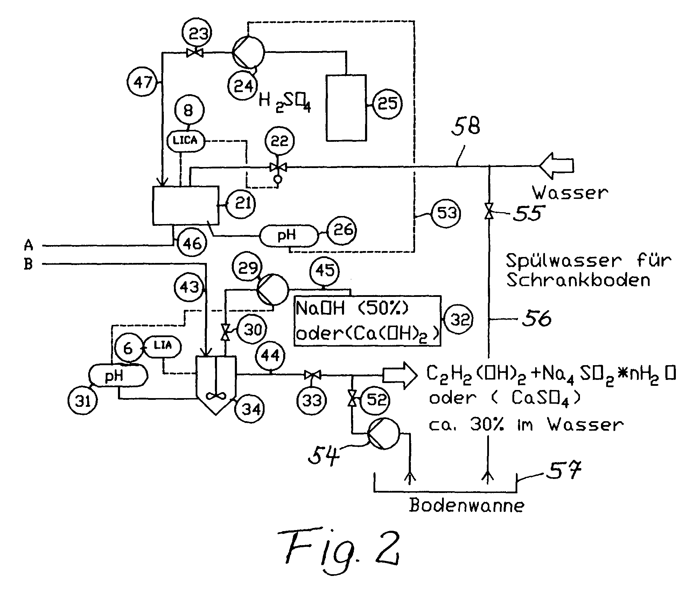

Figur 2      den zweiten Teil des Grundfließbildes der Vorrichtung ab den ankommenden Leitungen A-B aus Figur 1.

Bevorzugtes Ausführungsbeispiel der Erfindung:

Über eine Leitung E, in der ein Ventil 1 angeordnet ist, wird Ethylenoxid E, über eine Leitung F, in der ein Ventil 2 sich befindet, Frischluft F einer Sterilisationskammer 5 zugeführt. Gleichzeitig wird die Sterilisationskammer 5 über eine Kammerheizung 3, 4 vorzugsweise mittels Wasser, in bekannter Weise beheizt. Für die Zufuhr von Ethylenoxid E sowie für die Frischluftzufuhr F existieren mehrere, im Stand der Technik bekannte Verfahren, für die Frischluftzufuhr F auch in Verbindung mit Frischluftfiltern.

In der Sterilisationskammer 5, die in Größen von etwa 0,05 bis 70 m$^3$ in Gebrauch sind, wird das Sterilisiergut normalerweise mit Ethylenoxid in Gaskonzentrationen von 600 bis 1000 g/m$^3$ während einer vorgegebenen Zeit begast. Um eine gute Durchmischung des Ethylenoxids zu gewährleisten, wird ca. alle 10 Minuten die Gasatmosphäre umgewälzt.

Der Aufbau der Vorrichtung und somit des Gas-Umwälzkreislaufes besteht im wesentlichen aus der Sterilisationskammer 5, die über eine Ableitung 35, ein in dieser Leitung liegendes Ventil 9, die Vakuumpumpe 10, eine Leitung 39 und ein in dieser Leitung liegendes Ventil 11 mit einem Gaswäscher 15 verbunden ist, dessen Ausgang über eine Leitung 40, ein in dieser Leitung liegendes Ventil 16, einen Säureabscheider 17, eine Zuleitung 38 und ein in dieser Zuleitung liegendes Ventil 18 nach dem Säureabscheider 17 wiederum in die Sterilisationskammer 5 zurückgeführt ist. Dieser Gas-Umwälzkreislauf kann nach außen hin völlig geschlossen werden.

Vor der Durchführung der Begasung wird die Sterilisationskammer 5 mittels einer Vakuumpumpe mit Unterdruck beaufschlagt, vorzugsweise zwischen ca. 3000 bis 5000 Pascal, so daß anschließend das Ethylenoxid für die Sterilisation zugeführt werden kann. Im gezeigten Beispiel dient als Vakuum-und Umwälzpumpe eine Pumpe 10, die somit beide Funktionen in sich vereinigt.

Zur Umwälzung der Gasatmosphäre der Sterilisationskammer 5 dient ein Leitungsbypass 37 mit Ventil 14, der zwischen den Leitungen 39 und 40 jeweils vor den Ventilen 11 und 16 angeordnet ist. Zur Umwälzung der Gasatmosphäre sind die Ventile 9, 14 und 18 geöffnet, die Ventile 11 und 16 hingegen geschlossen. Auf diese Weise kann die Vakuumpumpe 10 das Gasgemisch über die Leitungen 35, 37, 38 und die Sterilisationskammer 5 umwälzen.

Parallel zur Sterilisationskammer 5 kann zwischen die Leitungen 35 und 38 vor der Pumpe 10 ein explosionsgeschütztes Umluftgebläse, mit vor- und nachgeordneten Ventilen, vorgesehen sein, insbesondere wenn als Vakuumpumpe eine Wasser-ringpumpe 10 eingesetzt wird. Zur Umwälzung der Gasatmosphäre mittels des Umluftgebläses während des Sterilisationsvorganges werden die Ventile 9 und 18 in den Leitungen 35 bzw. 38 geschlossen und nur die Ventile des Umluftgebläses geöffnet.

Normalerweise werden im Stand der Technik aus Sicherheitsgründen Drehschieber- oder Kolbenpumpen eingsetzt, weil bei einem Kontakt des Ethylenoxids zwischen dem Wasser des Wasserrings der Wasseringpumpe und dem Ethylenoxid dieses zu erheblichen Anteilen im Wasser in Lösung geht, wodurch Entsorgungsprobleme entstehen, obwohl Wasserringpumpen außerordentlich betriebssicher sind. Erfindungsgemäß wird dieser Nachteil in einen Vorteil umgemünzt, weil beim erfindungsgemäßen Einsatz einer Gaswäsche Dünnsäure, vorzugsweise 5%ige Schwefelsäure, für das Umlaufwasser des Wasserrings verwendet wird und durch den Kontakt des Ethylenoxids mit Wasserstoffionen, die als Katalysator wirken, Ethylenoxid spontan in Ethylenglykol umgewandelt wird und damit bereits zu einem Teil das Ethylenoxid dem Abgas entzogen wird.

Zur Vorbereitung der Sterilisationskammer 5 auf die Begasung wird diese nach dem Befüllen mit Sterilisiergut bis zur Leistunggrenze der Vakuumpumpe 10 (ca. 40-50 mbar) evakuiert. Dazu werden die Ventile 1, 2, 11, 16, 18 geschlossen und die Ventile 9, 14 geöffnet; ebenso wird ein in einer Abgasleitung 42 liegendes Ventil 19 geöffnet und die Vakuumpumpe 10 in Betrieb genommen und die Luft über die Leitungen 37 und 42 evakuiert. Die Leitungen 37 und 40 kreuzen sich mit der Leitung 42 mittels Doppel-T-Rohren, wobei sich vor der Kreuzung in der Leitung 40 das Ventil 16 und danach das Ventil 18 befindet. Ist die Evakuierung der Sterilisationskammer 5 abgeschlossen, werden die Ventile 9, 14, 19, geschlossen und die Vakuumpumpe 10 abgestellt. Danach erfolgt in bekannter Weise die Zudosierung von Ethylenoxid über die Leitung E, gegebenenfalls auch von Wasserdampf über das Ventil 1.

Nach Begasung des Sterilisiergutes in der Sterilisationskammer 5 über einen vorgegebenen Zeitraum wird das Ethylenoxid kontrolliert aus der Kammer 5 abgeführt und ist zu entsorgen. Hierfür dient als Kern des Gas-Umwälzkeislaufes ein Gaswäscher 15, in den über das Ventil 11 und die Leitung 39 das Ethylenoxid mittels der Vakuumpumpe 10 gefördert wird, die dazu wieder in Betrieb gesetzt wird. Die Ableitung 40 des Gaswäschers 15 führt über die Ventile 16 und 18 in den Dünnsäureabscheider 17, auf den die Leitung 38 folgt, die in die Sterilisationskammer 5 mündet. Bei geöffneten Ventilen 9, 11, 16, 18 existiert somit zur Gaswäsche des Ethylenoxids ein Entsorgungskreislauf zwischen Sterilisationskammer 5 - Vakuumpumpe 10 - Gaswäscher 15 - Dünnsäureabscheider 17 - Sterilisationskammer 5, ohne daß eine Öffnung der Anlage zur Außenluft erfolgt. Während der Gaswäsche, deren Gaskreislauf durch die Vakuumpumpe 10 aufrecht erhalten wird, sind sämtliche nach außerhalb der Anlage führenden Ventile, wie Ventile 1, 2, 14, 19, geschlossen.

Vorzugsweise ist der Gaswäscher 15 größenmäßig derart ausgelegt, daß das Ethylenoxid aus der Sterilisationskammer 5 um eine definierte Höhe h unter der Oberfläche der im Gaswäscher 15 befindlichen Dünnsäure von unten feinblasig eingeblasen wird, die vorzugsweise 5%-ige Schwefelsäure ist. Die Höhe berechnet sich nach dem Durchsatz des Gasstromes abhängig von der Kammergröße und der Durchströmzeit der einzelnen Gasblasen durch die Dünnsäure, woraus sich eine bestimmte Durchströmungslänge berechnet, die mindestens bei 500 mm Höhe für kleine Anlagen liegt.

Durch die große Phasengrenzfläche zwischen den Abgasblasen des Ethylenoxids und der Dünnsäure reagiert das im Abgas enthaltene Ethylenoxid mit den in der Dünnsäure enthaltenen $H^+$-Ionen als Katalysator und dem Wasser nahezu vollständig zu Ethylenglykol gemäß der Formel:

$$C_2H_4O + H_2O \xrightarrow{2\ H^+\ SO_4^-} C_2H_4(OH)_2$$

Da der Flüssigkeitsspiegel der Dünnsäure im Gaswäscher 15 für die weitgehende Umwandlung des Ethylenoxids zu Ethylenglykol nur höchstens 2 m über der Ausblasstelle liegen muß, arbeitet die Vakuumpumpe nur gegen eine Druckdifferenz von 200 mbar, wodurch nahezu die maximale Förderleistung relaisierbar ist. Dadurch wird vorteilhaft erreicht, daß das Auswaschen des Ethylenoxids praktisch in der gleichen Zeit erfolgt, wie nach dem Stand der Technik für 2 bis 3 Spülzyklen benötigt wird.

Während der Gaswäsche kann auf zweierlei Weise verfahren werden. Entweder wird in diskontinuierlicher Weise die während eines vollständigen Sterilisationsvorganges, unter Umständen mit mehreren Umwälzzyklen, sich verbrauchende Dünnsäure im Gaswäscher nicht ersetzt und das sich bildende Ethylenglykol nicht entzogen, so daß sämtliche nach außerhalb der Anlage führenden Ventile während des vollständigen Sterilisationsvorganges geschlossen sind, sondern erst nach Beendigung des Sterilisationsvorganges und des kompletten Arbeitsablaufes. Erst danach wird die verbrauchte Dünnsäure ersetzt und das Ethylenglykol entsorgt. Oder es wird in kontinuierlicher Weise während der Gaswäsche die verbrauchte Dünnsäure im Gaswäscher kontinuierlich nachgegeben und das Ethylenglykol entweder ebenfalls kontinuierlich während der Gaswäsche oder auch diskontinuierlich nach sämtlichen Gaswaschvorgängen entsorgt.

Aus Sicherheitsgründen sollte die Zeit für das Auswaschen des Ethylenoxids so gewählt werden, daß ca. die 3- bis 4-fache Zeit für die Evakuierung der Sterilisationskammer 5 als Normalbetrieb für die Gaswäsche als Richtwert genommen wird, um auchletzte Reste an Ethylenoxid sicher zu entsorgen.

Durch das Auswaschen des Ethylenoxids aus dem Kammergas und die Umwandlung desselben in Ethylenglykol wird laufend der Innendruck in der Sterilisationskammer 5 und im Gaskreislauf vermindert. Nach Abschluß der Gaswäsche werden die Ventile 11, 16, und 18 geschlossen, die Ventile 14 und 19 geöffnet sowie die Vakuumpumpe 10 angeschaltet und auf diese Weise die Sterilisationskammer 5 über die Abgasleitung 42 evakuiert. Aufdiese Weise kann das vorzugsweise verwendete, für die Umwelt unschädliche, in höheren Konzentrationen jedoch u.U. gesundheitlich bedenkliche Inertgas abgeführt werden. Nach Abschluß der Evakuierung werden die Ventile 14 und 19 geschlossen, die Vakuumpumpe 10 abgestellt und auch die Ventile 9, 11, 16 geschlossen; über das Ventil 2 wird der Sterilisationskammer 5 Frischluft bis zum Druckausgleich zugeführt. Danach kann die Sterilisationskammer 5 geöffnet und das Sterilisergut entnommen werden.

Der Dünnsäureabscheider 17 in den Leitungen 38-40 zwischen den Ventilen 16 und 18 dient zum Abscheiden fein dispergierter Flüssigkeitströpfchen aus dem umgewälzten Abgas, das den Gaswäscher 15 an seinem Ausgang über die Leitung 40 verläßt. Die auf diese Weise zurückgewonnene Dünnsäure wird über eine Leitung 41, ein geöffnetes Ventil 12 und die Rückschlagklappe 13 in den Gaswäscher 15 zurückgeleitet.

Im Gaswäscher 15 verliert die Dünnsäure beim Ablauf der Gaswäsche ihre normale Reaktionsfähigkeit, wenn sie bis ca. 35% mit Ethylenglykol angereichert ist. Pro 1 kg entsorgten Ethylenoxids entstehen ca. 1,41 kg Ethylenglykol, womit je 1 kg Ethylenoxid ca. 4,6 l Waschflüssigkeit nach Ende des Waschvorganges aus dem Gaswäscher 15 über eine Leitung 43 mit einem Ventil 28 einer an sich bekannten Neutralisations- und Abscheideeinheit 34 zugeleitet werden müssen. Durch eine pH-Wert-Regeleinheit 31 erfolgt eine pH-Wert-geregelte Zudosierung von Kalziumhydroxid aus einer Dosiereinrichtung 32 über eine Leitung 45, in der eine Pumpe 29 angeordnet ist, wodurch innerhalb der Neutralisations- und Abscheideeinheit 34 Gips entsteht, der sich in der Neutralisations- und Abscheideeinheit 34 absetzt und von dort abgezogen werden kann. Die entstandene 30%ige Ethylenglykollösung am Ausgang der Neutralisations- und Abscheideeinheit 34 kann über ein Ventil 33 einer Leitung 44 entnommen und einer Weiterverarbeitung zugeführt werden, wobei daraus extern wiederum Ethylenoxid gewonnen werden kann.

Zur Versorgung des Gaswäschers 15 mit Dünnsäure dient eine an sich bekannte Dünnsäureeinheit 21, die über eine Leitung 46 und ein Ventil 20 an den Gaswäscher 15 angeschlossen ist. Durch Zufuhr von Wasser über ein Ventil 22 und von Schwefelsäure aus einem Behälter 25, die mittels einer Pumpe 24 über ein Ventil 23 über eine Leitung 47 der Dünnsäureeinheit 21 aufgegeben wird, an die gleichzeitig eine pH-Wert-Regeleinheit 26 angeschlossen ist, wird pH-Wertgeregelte 5%-ige Schwefelsäure (Dünnsäure) gemischt. Durch die Dünnsäureeinheit 21 wird flüssigkeitsstandgeregelt genau die Menge an Dünnsäure pro Zeiteinheit dem Gaswäscher 15 zugeführt, die diesem vorher durch Bildung von Ethylenglykol aus Ethylenoxid pro Zeiteinheit über ein Regelventil 28 zur Neutralisation und Entsorgung des Ethylenglykols entzogen wurde.

Die Bezugsziffern 6, 7, 8, 8' bezeichnen Füllstandsregler, die zur Regelung der Füllstände in der Neutralisations- und Abscheideeinheit 34, dem Gaswäscher 15, der Dünnsäureeinheit 21 sowie dem Dünnsäureabscheider 17 dienen.

Die gesamte Anlage ist vorzugsweise in einem Schrank oder Skid angeordnet, der eine säurefeste Bodenwanne 57 aufweist zum Auffangen von flüssigen Verbrauchsmaterialien. Über eine Leitung 56 und ein Ventil 55 kann Spülwasser in die Bodenwanne 57 geleitet werden. Über ein Ventil 52 und eine Pumpe 54 kann gleichermaßen aus der Bodenwanne 57 das Abwasser in die Leitung 44 eingespeist und nach außen abgegeben und entsorgt werden.

Gewerbliche Anwendbarkeit:

Das Verfahren und die Vorrichtung sind insbesondere zur Sterilisation von mehrfachgenutzten Medikalgütern in Krankenhäusern, Altenheimen, Labors u.a. einsetzbar, in denen Sterilisatoren eingesetzt werden, die mit dem Gas Ethylenoxid zur Sterilisation betrieben werden. Die Nützlichkeit des Verfahren und der Vorrichtung besteht insbesondere darin, daß eine katalytische Verbrennung, wie beim Stand der Technik, entfällt, weil der Gaskreislauf bis zur praktisch nicht mehr meßbaren Absorption des Ethylenoxids betrieben werden kann, so daß die Abluft praktisch keine meßbare Menge an Ethylenoxid mehr enthält. Das Abgasvolumen besteht nur noch aus einem Bruchteil der Abgasmenge gegenüber den herkömmlichen Verfahren.

Liste der Bezugszeichen:

| | |
|---|---|
| 1, 2, 6, 11, 12, 14, 16, 18 19, 20, 22, 23, 30, 33, 52, 55 | Ventile |
| 3, 4 | Kammerheizung |
| 5 | Begasungskammer |
| 7, 8, 9 | Füllstandsregler |
| 10 | Vakuumpumpe (Wasserringpumpe) |
| 13 | Rückschlagklappe |
| 15 | Gaswäscher |
| 17 | Dünnsäureabscheider |
| 21 | Dünnsäureeinheit |
| 24 | Pumpe |
| 25 | Säurebehälter |
| 26 | pH-Wert-Regeleinheit |
| 28, 49 | Regelventil |
| 29, 54 | Pumpe |
| 31 | pH-Wert-Regeleinheit |
| 32 | Dosiereinrichtung |
| 34 | Neutralisations- und Abscheideeinheit |
| 35 | Ableitung |
| 38 | Zuleitung |
| 36, 37, 39, 40, 41, 42 43, 44, 45, 46, 47, 53, 56, 58 | Leitungen |

| 48 | Gasbehälter für Inertgas |
| 50 | Druckregler |
| 57 | Bodenwanne |

## Patentansprüche

1. Verfahren zum Entsorgen des Gases Ethylenoxid nach erfolgter Begasung von zu sterilisierendem Gut innerhalb einer druckdicht verschließbaren Sterilisationskammer (5), unter Verwendung einer an die Sterilisationskammer (5) angeschlossenen Pumpe (10), die das Ethylenoxid nach der Begasung einem mit Säure arbeitenden Gaswäscher (15) zuführt, in welchem das Ethylenoxid in Ethylenglykol umgewandelt wird, wobei Sterilisationskammer (5), Pumpe (10) und Gaswäscher (15) einen geschlossenen Gaskreislauf für das Ethylenoxidgas bilden, innerhalb des Gaskreislaufes die Pumpe (10) das Ethylenoxid aus der Sterilisationskammer (5) kontinuierlich dem Gaswäscher (15) zuführt, dessen Abgase zurück der Sterilisa-tionskammer (5) aufgegeben werden, die über eine Kammerheizung (3,4) beheizt wird, wobei das im Gaswäscher (15) entstehende Ethylenglykol kontinuierlich oder diskontinuierlich aus dem Gaswäscher (15) abgezogen und dafür dem Gaswäscher (15) entsprechend dem Verbrauch an Säure kontinuierlich oder diskontinuierlich weitere Säure zugegeben wird und durch das Auswaschen des Ethylenoxids aus dem Kammergas und die Umwandlung desselben in Ethylenglykol der Innendruck in der Sterilisationskammer (5) und im Gaskreislauf laufend vermindert wird, und daß dieser Gas-Kreisprozeß solange aufrechterhalten wird, bis der Ethylenoxid-anteil des im Gaskreislauf verbliebenen umgewälzten Gases unter eine vorgege-bene Konzentration gefallen ist und danach die Sterilisationskammer (5) belüftet wird.

2. Verfahren nach Anspruch 1, dadurch gekennzeichnet,
daß die Sterilisation innerhalb der Sterilisationskammer (5) mit einem Unterdruck gegenüber dem Athmosphärendruck betrieben wird, vorzugsweise mit einem Druck von 0,5 bar.

3. Verfahren nach Anspruch 1, dadurch gekennzeichnet,
daß die Pumpe (10) sowohl eine Vakuum- als auch eine Umwälzpumpe ist, die die Sterilisationskammer (5) zur Einleitung des Begasungsvorgangs leerpumpt als auch nach der Sterilisation den Gaskreislauf ingangsetzt und aufrechterhält, insbesondere die Pumpe (10) eine Wasserringpumpe ist, deren Wasserring vorzugsweise Dünnsäure aus 5%iger Schwefelsäure zugesetzt wird.

4. Verfahren nach Anspruch 1, dadurch gekennzeichnet,
daß das Ethylenoxid aus der Sterilisationskammer 5 um eine definierte Höhe h unter der Oberfläche der im Gaswäscher 15 befindlichen Dünnsäure von unten feinblasig eingeblasen wird, die vorzugsweise 5 %-ige Schwefelsäure ist, wobei die Höhe h mindestens 500 mm beträgt.

5. Vorrichtung zur Durchführung des Verfahrens nach Anspruch 1, dadurch gekennzeichnet,
daß eine Sterilisationskammer (5), ein Gaswäscher (15) und eine Vakuumpumpe (10) zum kontinuierlichen Zuführen des Ethylenoxids aus der Sterilisationskammer (5) in den Gaswäscher (15), über Leitungen (35,38,39,40) zu einem geschlossen Gaskreislauf zusammengeschlossen sind, daß die Sterilisationskammer (5) mittels Kammerheizung (3,4) beheizbar ist und der Gaswäscher (15) so mit einer Dünnsäureeinheit (21) verbunden ist, daß Ethylenglykol abgezogen und Säure zugegeben werden kann, daß die Pumpe (10) den Kreisprozeß solange aufrechterhalten kann, bis der Ethylenoxidanteil des im Gaskreislauf verbliebenen Gases unter eine vorgegebene Konzentration gefallen ist und daß die Sterilisationskammer belüftet werden kann.

6. Vorrichtung nach Anspruch 5, dadurch gekennzeichnet,
daß innerhalb des Gaskreislaufes (5,35,10,39,15,40,38) ein Säureabscheider (17) angeordnet ist, dessen ein Ausgang (41) über eine Rückschlagklappe (13) in den Gaswäscher (15) geführt ist.

7. Vorrichtung nach Anspruch 5 oder 6, dadurch gekennzeichnet,
daß die Vakuumpumpe (10) auch eine Umwälzpumpe ist zum Leerpumpen der Sterilisationskammer (5) zur Einleitung des Begasungsvorgangs als auch zum Ingangsetzen und Aufrechterhaltung des Gaskreislaufes nach der Sterilisation, wobei die Vakuumpumpe (10) gegebenenfalls über einen Bypass (37) mit der Sterilisationskammer (5) verbunden ist.

8. Vorrichtung nach Anspruch 7, dadurch gekennzeichnet,
daß die Vakuumpumpe eine Wasserring-Vakuumpumpe (10) ist, deren Wasserring mit Umlaufwasser betrieben wird, dem Dünnsäure, vorzugsweise 5%ige Schwefelsäure, zugesetzt ist.

9. Vorrichtung nach Anspruch 5, dadurch gekennzeichnet,
daß parallel zur Sterilisationskammer (5) ein Umluftgebläse angeordnet ist, welches das Ethylenoxid-Gasgemisch innerhalb der Sterilisationskammer (5) während des Sterilisationsprozesses umzuwälzen imstande ist.

**Claims**

1. Process for disposing of ethylene oxide gas after carrying out the gassing of articles to be sterilized inside a sterilization chamber (5) that can be sealed pressure-tight, using a pump that is connected to the sterilization chamber (5) and that conveys the ethylene oxide after the gassing to a gas scrubber (15) that functions with acid, in which the ethylene oxide is converted into ethylene glycol, whereby the sterilization chamber (5), the pump (10) and the gas scrubber (15) form a closed gas circuit for the ethylene oxide gas, whereby, within the gas circuit, the pump (10) continuously feeds the ethylene oxide from the sterilization chamber (5) to the gas scrubber (15) whose exhaust gases are conveyed back to the sterilization chamber (5) which is heated by means of a chamber heater (3, 4), whereby the ethylene glycol being formed in the gas scrubber (15) is continuously or discontinuously withdrawn from the gas scrubber (15) and in exchange, additional acid is continuously or discontinuously fed into the gas scrubber (15) corresponding to the consumption of acid and, through the scrubbing of the ethylene oxide from the chamber gas and the conversion of said ethylene oxide into ethylene glycol, the internal pressure in the sterilization chamber (5) and in the gas circuit is constantly reduced) and in that this gas circulation process is maintained until the ethylene oxide fraction of the circulated gas remaining in the gas circuit has fallen below a pre-defined concentration and then the sterilization chamber (5) is vented.

2. Process according to Claim 1, characterized in that
the sterilization inside the sterilization chamber (5) is carried out at a negative pressure with respect to the atmospheric pressure, preferably at a pressure of 0.5 bar.

3. Process according to Claim 1, characterized in that
the pump (10) is a vacuum pump as well as a circulation pump that pumps the sterilization chamber (5) empty in order to initiate the gassing procedure and it also starts up and maintains the gas circulation after the sterilization, in particular the pump (10) is a water ring pump to whose water ring preferably dilute acid from 5%-sulfuric acid is added.

4. Process according to Claim 1, characterized in that
the ethylene oxide from the sterilization chamber (5) is injected from below in the form of fine bubbles at a defined height **h** under the surface of the dilute acid, which is preferably 5%-sulfuric acid, present in the gas scrubber (15), whereby the height **h** is at least 500 mm.

5. Device for executing the process according to Claim 1, characterized in that
a sterilization chamber (5), a gas scrubber (15) and a vacuum pump (10) for the continuous feed of the ethylene oxide from the sterilization chamber (5) into the gas scrubber (15) are linked via lines (35, 38, 39, 40) to form a closed gas circuit, in that the sterilization chamber (5) can be heated by means of a chamber heater (3, 4), and in that the gas scrubber (15) is connected with a dilute acid unit (21) in such a way that ethylene glycol can be withdrawn and acid can be added, in that the pump (10) can maintain the circulation process until the ethylene oxide fraction of the gas remaining in the gas circuit has fallen below a pre-defined concentration and in that the sterilization chamber can be vented.

6. Device according to Claim 5, characterized in that,
within die gas circuit (5, 35, 10, 39, 15, 40, 38), there is an acid separator (17) whose one output (41) leads via a return valve (13) into the gas scrubber (15).

7. Device according to Claim 5 or 6, characterized in that
the vacuum pump (10) is also a circulation pump for pumping empty the sterilization chamber (5) in order to initiate the gassing procedure and also to start up and maintain the gas circulation after the sterilization, whereby the vacuum pump (10) is optionally connected to the sterilization chamber (5) via a bypass (37).

8. Device according to Claim 7, characterized in that
the vacuum pump is a water ring vacuum pump (10) whose water ring is operated with circulating water to which dilute acid, preferably 5%-sulfuric acid, is added.

9. Device according to Claim 5 characterized in that
parallel to the sterilization chamber (5), there is a circulation fan that is capable of circulating the ethylene oxide gas mixture inside the sterilization chamber (5) during the sterilization process.

**Revendications**

1. Procédé d'élimination d'oxyde d'éthylène gazeux utilisé pour stériliser des objets à l'intérieur d'une chambre de stérilisation (5) pouvant être fermée en tenant la pression, procédé faisant appel à une pompe (10) reliée à la chambre de stérilisation (5) qui conduit l'oxyde d'éthylène, après son usage pour stérilisation, dans un épurateur de gaz (15) fonctionnant à l'acide dans lequel l'oxyde d'éthylène se transforme en éthylène glycol, la chambre de stérilisation (5), la pompe (10) et l'épurateur de gaz (15) formant un système de circulation du gaz d'oxyde d'éthylène en circuit fermé à l'intérieur duquel la pompe (10) fait passer en continu l'oxyde d'éthylène de la chambre de stérilisation (5) à l'épurateur de gaz (15) dont les gaz qui s'échappent sont à nouveau éconduits dans la chambre de stérilisation (5) chauffée au moyen d'un chauffage (3, 4), l'éthylène glycol produit dans l'épurateur de gaz (15) étant extrait de façon continue ou discontinue de l'épurateur de gaz (15), en échange de quoi, en fonction de la consommation d'acides, un nouvel apport d'acides est ajouté en continu ou en discontinu dans l'épurateur de gaz (15) et, de par le lavage de l'oxyde d'éthylène provenant de la chambre de stérilisation et la transformation de ce dernier en éthyle glycol, la pression interne dans la chambre de stérilisation (5) et dans le circuit de circulation du gaz diminue en permanence, et du fait que ce processus de circulation du gaz étant maintenu jusqu'à ce que la teneur en oxyde d'éthylène du gaz brassé qui reste dans le circuit de circulation du gaz tombe au-dessous d'une concentration définie, après quoi la chambre de stérilisation (5) est ventilée.

2. Procédé selon la revendication 1, caractérisé en ce que
la stérilisation se produit dans la chambre de stérilisation (5) sous dépression par rapport à la pression atmosphérique, de préférence à une valeur de vide de 0,5 bar.

3. Procédé selon la revendication 1, caractérisé en ce que
la pompe (10) est une pompe à vide aussi bien qu'une pompe de circulation qui établit le vide dans la chambre de stérilisation (5) avant que le processus de stérilisation ne soit amorcé, et qui enclenche et maintient aussi bien la circulation du gaz en circuit fermé, une fois que la stérilisation est achevée. La pompe (10) est notamment une pompe à anneau d'eau, anneau d'eau auquel est ajouté de préférence un acide dilué qui est de l'acide sulfurique concentré à 5%.

4. Procédé selon la revendication 1, caractérisé en ce que
l'oxyde d'éthylène de la chambre de stérilisation (5) est pulvérisé à partir du bas à une hauteur définie h au-dessous de la surface de l'acide dilué qui se trouve dans l'épurateur de gaz (15), l'acide dilué étant de préférence de l'acide sulfurique à 5%, et la hauteur h devant être de 500 mm au moins.

5. Dispositif pour la mise en oeuvre du procédé selon la revendication 1, caractérisé en ce que
une chambre de stérilisation (5), un épurateur de gaz (15) et une pompe à vide (10) sont reliés par des conduites (35,38,39,40) et constituent un circuit de circulation du gaz en système clos qui fait passer en continu l'oxyde d'éthylène de la chambre de stérilisation (5) à l'épurateur de gaz (15), en ce que la chambre de stérilisation (5) est chauffée par un chauffage (3,4) et que l'épurateur de gaz (15) est relié à un agrégat d'acide dilué (21), de sorte que glycol éthylène peut être extrait et qu' un nouvel apport d'acide peut se faire, en ce que la pompe (10) maintient le process de circulation en cours jusqu'à ce que la teneur en oxyde d'éthylène contenue dans le gaz restant dans le circuit tombe au-dessous d'une concentration définie, et en ce que la chambre de stérilisation peut être ventilée.

6. Dispositif selon la revendication 5, caractérisé en ce que
à l'intérieur du circuit de circulation du gaz (5,35,10,39,40,38) est agencé un séparateur d'acides (17) dont l'une des conduites de sortie (41) mène à l'épurateur de gaz (15) via un clapet de non-retour (13).

7. Dispositif selon l'une des revendications 5 ou 6, caractérisé en ce que
la pompe à vide (10) est également une pompe de circulation qui permet aussi bien de faire le vide dans la chambre de stérilisation (5) afin de pouvoir amorcer le processus de stérilisation que d'enclencher et de maintenir la circulation du gaz en circuit après la stérilisation, la pompe à vide (10) étant le cas échéant reliée à la chambre de stérilisation (5) au moyen d'une conduite à by-pass (37).

8. Dispositif selon la revendication 7, caractérisé en ce que

9

la pompe à vide est une pompe à anneau d'eau (10) dont l'anneau d'eau est activé par l'eau en circulation à laquelle est rajouté de l'acide dilué, de préférence de l'acide sulfurique à 5%.

9. Dispositif selon la revendication 5, caractérisé en ce que
parallèlement à la chambre de stérilisation (5) est installée une soufflerie qui est en mesure de brasser le mélange gazeux d'oxyde d'éthylène à l'intérieur de la chambre de stérilisation (5) au cours du processus de stérilisation.

Fig. 1

Fig. 2